# EUROPEAN PATENT APPLICATION

(11) **EP 4 817 522 A1**
(43) Date of publication of application: **07.10.2026**
(21) Application number: 26170225.2
(22) Date of filing: 01.04.2026
(51) Int. Cl.: A61B 5/355, A61B 5/36, A61B 5/00, A61B 5/367, A61B 5/319

(54) **DETERMINING REPOLARIZATION WAVES USING A SIMULATED HEART MODEL**

(30) Priority: 02.04.2025 US 202563782332 P; 18.03.2026 US 202619571435
(71) Applicant: Biosense Webster (Israel) Ltd., 2066717 Yokneam (IL)
(72) Inventor: BOTZER, Lior, 2066717 Yokneam (IL); MALKI, Guy, 2066717 Yokneam (IL); BAR-TAL, Meir, 2066717 Yokneam (IL)
(74) Representative: Carpmaels & Ransford LLP

(57) **Abstract**

An example method may use simulated data a virtual heart model to measure characteristics including cell potential and determine repolarization timing, while collecting accurate, simulated intracardiac signals. The determined characteristics (e.g., repolarization timing) may be annotated for reference. The collected signals combined with the annotated timing may be used to train an AI model. The AI model may be configured to determine characteristics such as repolarization timing based on input of one or more intracardiac signal(s). The AI model may be tested by inputting a dataset comprising intracardiac signals from other heart models and receiving a predicted ARP from the AI model. The predicted ARP may be compared with known ARPs to determine an accuracy of the AI model. The other heart models may be associated with heart conditions and/or complications, such as tachycardias.

## Description

### CROSS-REFERENCE TO RELATED APPLICATION

This application is a Nonprovisional of and claims the benefit of Provisional Application No. 63/782,332, filed April 2, 2025, which is hereby incorporated by reference in its entirety for any and all purposes.

### BACKGROUND

Detecting an atrial repolarization wave may help diagnose atrial tachycardias and/or atrial fibrillation. However, detecting a repolarization wave in an intracardiac signal is a complicated task. A repolarization wave may often be unnoticeable or masked by a low signal-to-noise ratio (SNR) such as respiration and/or by other far field signals.

Therefore, other means of identification of the repolarization wave is required. Furthermore, perhaps the more interesting question is related to the repolarization state of the cells and the recorded electrode potential is just a surrogate for this and not exact measure.

### SUMMARY

It is to be understood that both the following general description and the following detailed description are exemplary and explanatory only and are not restrictive. Methods and systems for detecting repolarization waves using a simulated heart model are described.

A system of one or more computers can be configured to perform particular operations or actions by virtue of having software, firmware, hardware, or a combination of them installed on the system that in operation causes or cause the system to perform the actions. One or more computer programs can be configured to perform particular operations or actions by virtue of including instructions that, when executed by data processing apparatus, cause the apparatus to perform the actions.

In one general aspect, methods may include determining first data indicative of one or more of unipolar intracardiac signals of a heart, a bipolar intracardiac signals of a heart, or multipolar intracardiac signals of a heart. Methods may also include determining second data indicative of one or more action potential signals of a heart having an action recovery period (ARP). Methods may furthermore include training an artificial intelligence (AI) model based on the first data and the second data, where the AI model is configured to determine a predicted ARP based on one or more of unipolar intracardiac signals of a heart, a bipolar intracardiac signals of a heart, or multipolar intracardiac signals of a heart. Methods may in addition include receiving third data indicative of one or more of unipolar intracardiac signals of a heart, a bipolar intracardiac signals of a heart, or multipolar intracardiac signals of a heart. Methods may moreover include determining a predicted ARP from the third data. Other embodiments of this aspect include corresponding computer systems, apparatus, and computer programs recorded on one or more computer storage devices, each configured to perform the actions of the methods.

These and other features and advantages are described in greater detail below.

### BRIEF DESCRIPTION OF THE DRAWINGS

Some features are shown by way of example, and not by limitation, in the accompanying drawings. In the drawings, like numerals reference similar elements.
FIG. 1 shows an example image of an example model described herein.
FIG. 2 shows an example model described herein.
FIG. 3 shows an example graph described herein.
FIG. 4 shows an example method for training an example model disclosed herein.
FIG. 5 shows an example method for testing an example model disclosed herein.
FIG. 6 illustrates a flowchart of illustrates a flowchart of an example method in accordance with the present disclosure.
FIG. 7 illustrates a flowchart of an example method in accordance with the present disclosure.
FIG. 8 illustrates a flowchart of an example method in accordance with the present disclosure.

The accompanying drawings show examples of the disclosure. It is to be understood that the examples shown in the drawings and/or discussed herein are non-exclusive and that there are other examples of how the disclosure may be practiced.

### DETAILED DESCRIPTION

The present disclosure relates to detection of one or more repolarization wave(s) from a simulated heart model comprising cell potential and simulated extra cell potentials.

The present disclosure relates to systems and/or methods for modeling repolarization in heart cells. An example method may use a virtual heart model comprising a plurality of cells (e.g., 10 million), cells interaction, conduction system, the myocardium layers and the heart anatomy and pathology to simulate heart activity. The model may be configured to exhibit various simulated heart activities under various conditions. By leveraging such a heart model(s), one may generate any number of simulated scenarios and may gather output data relating to a modeled scenario. For example, methods of the present disclosure may combine electrophysiology and artificial intelligence (AI) to accurately study and track cell repolarization and its effect \on a measured Intracardiac signal.

In the healthy heart, cardiac muscle cells are electrically stimulated to contract in a sequential, synchronized manner, the propagation starting in the sinus node in the right atrium and spreading through the heart as each cell stimulates neighboring cells. The activation of each muscle cell is followed by a period in which the cell is incapable of being stimulated again. This period is called the refractory period, and it functions to ensure a smooth contraction of the muscle and the efficient flow of blood through the chambers of the heart. In a patient with cardiac arrhythmia, the propagation of the electrical stimulation of the muscle does not proceed as it should. The heart rate may be too fast or too slow, or the electrical stimulation of the heart may progress through an abnormal pathway, initiated elsewhere or become self-sustained (like in Macro Reentry or Atrial fibrillation). The abnormal pathways may be fixed or they may be functional, i.e., with no fixed circuit or focus they can occur in atrial or ventricular chambers or both it can be of epi or endo origin or both and may include other structures outside the 4 main chambers

For identifying the electrical activation sequence of the heart, the local electrical activity is acquired at a site within the patient's heart chamber using a steerable catheter, the position of which is assessed by transillumination images in which the heart chamber is not visible. Local electrical activation time, measured as time elapsed from a common reference event in the cardiac cycle to a fiducial point during the electrical systole, represents the local activation time (LAT) needed to construct the activation map data point at a single location.

Conventionally, to generate a complete activation map of the heart, several data points may be sampled. The catheter may be moved to a different location within the heart chamber and the electrical activation is acquired again, the mapping electrode location is used to establish and associate the LAT within the anatomy. The activation map generated may be used to identify the electrical pathway that is the cause of the patient's arrhythmia.

As an illustration, methods in accordance with the present disclosure may generate simulated data from a virtual heart model. Such data may include characteristics such as an electrical potential of the cells of the modelled heart and determine a timing of depolarization and also repolarization of the cells (e.g., recovery timing - the time it requires a cell to be ready for activation again), while collecting accurate simulated signals. The signals and corresponding recovery timing may be used to train an AI model to learn how the recovery timing relates to a large set of recorded signals. As an example, a model (e.g., AI model) may be trained using one or more of unipolar intracardiac signals, bipolar intracardiac signals, or multipolar intracardiac signals and an action potential or group of action potentials together. The model may be trained on one or more data sets simulated using the heart model. The action potential output (e.g., the action potential of the cell as function of time) may be indicative an action recovery period (ARP) from the heart model's cell activity. As such, patterns in the one or more of unipolar intracardiac signals, bipolar intracardiac signals, or multipolar intracardiac signals may be correlated with the action potential, and particularly timing characteristics of the action potential such as the ARP or repolarization timing. Multiple timing parameters or characteristics of waveforms may be trained for detection such as the maximum AP, functions or derivations of the AP waveform (e.g., dv/dt, or percentage from Maximum AP value change) By developing an AI model in this way, the model may then receive one or more of unipolar intracardiac signals, bipolar intracardiac signals, or multipolar intracardiac signals without the benefit of the action potential, and may still predict a reliable ARP.

The AI model may be trained and/or tested on different heart models and datasets exhibiting the same or different design characteristics such as modelling age, size, biological conditions, or other characteristics of the heart. The AI model may be trained and/or tested on different heart models and datasets that involve heart conditions, with different rhythms, pacing and tachycardias. A predicted recovery timing determined by the AI model may be compared to the known recovery timing to check an accuracy of the AI model. The example method may use the heart model, physiological, anatomical, and/or physical modeling, and/or the AI model to detect accurately one or more repolarization wave(s) within intracardiac signals.

The example method may use the simulated data from the virtual heart model to measure cell potential and determine repolarization timing, while collecting accurate, simulated intracardiac signals. The determined repolarization timing may be annotated for reference. The collected signals combined with the annotated timing may be used to train the AI model. The AI model may be configured to determine repolarization timing based on input of one or more intracardiac signal(s). The AI model may be trained on a large set of intracardiac signals.

The AI model may be trained on unipolar intracardiac signals and known ARP. The known ARP may be determined by cell activity associated with the heart model. Data used to train the AI model may be augmented to simulate real heart conditions or activities. Augmenting the data used to train the AI model may comprise adding noise, respiration modulation, mechanical heartbeat position changes to the data and/or adding far field signals such as ventricle far field signals to the data. Once the AI model is trained, the AI model may be tested by inputting a dataset comprising intracardiac signals from other heart models and receiving a predicted ARP from the AI model. The predicted ARP may be compared with known ARPs to determine an accuracy of the AI model. The other heart models may be associated with heart conditions and/or complications, such as various tachycardias such as micro reentry, macro reentry, AFib, NSR or Pacing.

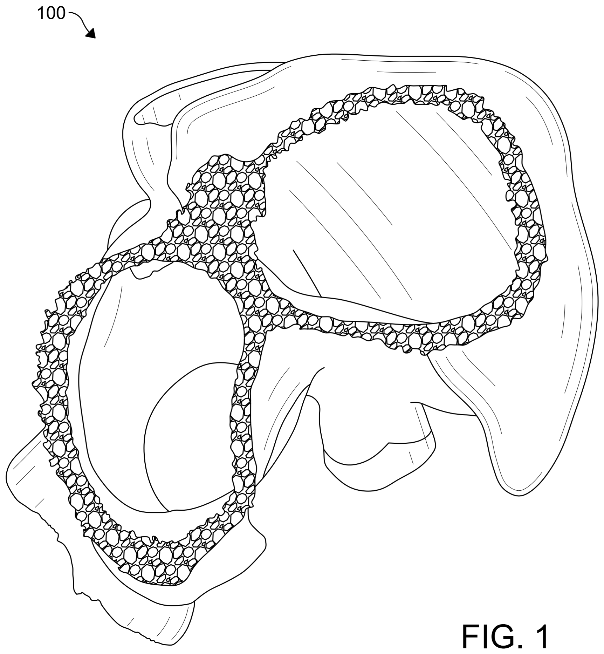

FIG. 1 shows an example image 100 of an example model described herein. The example model may simulate movement of a heart comprising a similar structure. The example model may comprise a plurality of simulated cells such as ten million or more simulated cells. The example model may simulate various waveform propagations. A state of action potential in the cells may be measured using the modelled heart or by actual measurement using a simulated electrode. Other simulations may be used, as described herein.

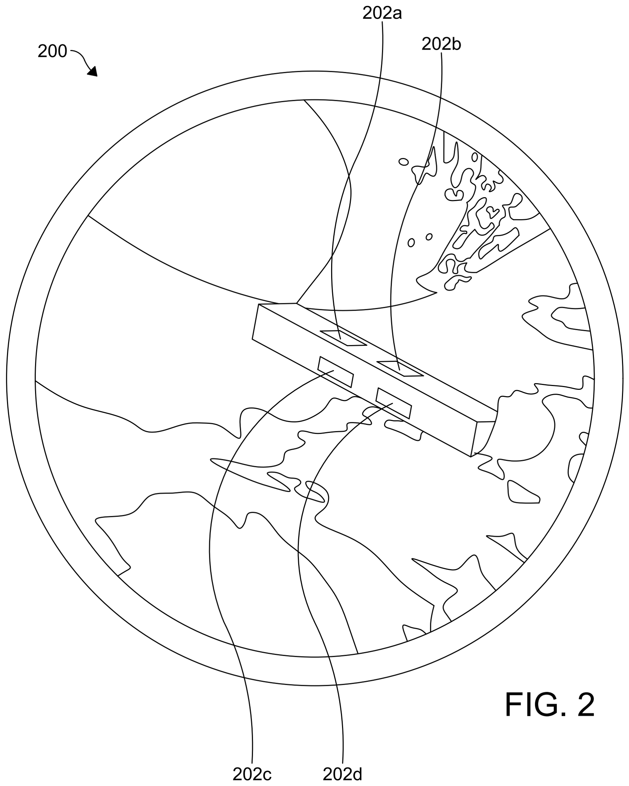

FIG. 2 shows an example model 200 described herein. As shown, the example model may comprise an electrode, or several electrodes (202a-202d), the model can also simulate the non-conductive element of catheter elements. The electrode may be used to generate signals used to create the model or supplement the modelled data. Signals and/or data represented by such signals may comprise various characteristics, including tissue characteristics, parameters of a cell or population of cells, characteristics of zones of interest of tissue, and/or other parameters that may be measured or modelled.

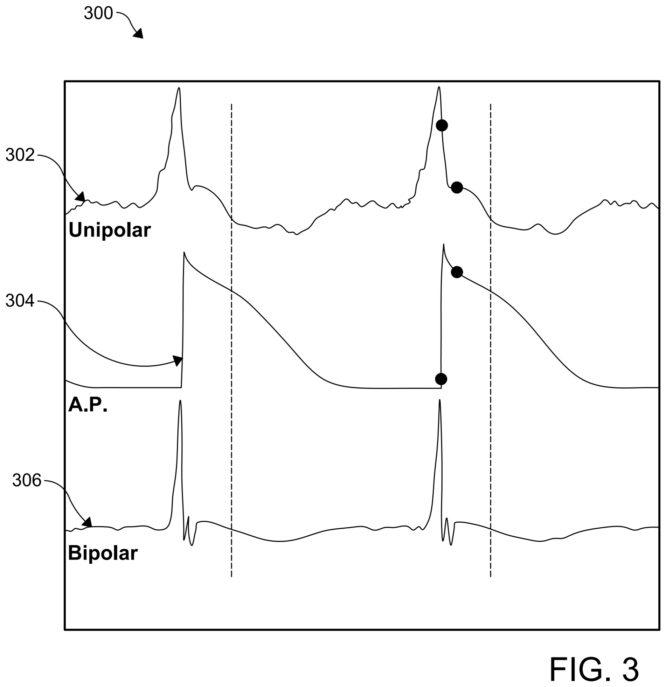

FIG. 3 shows an example graph 300 described herein. The example graph 300 may show a relationship between detected/measured or modelled signals. As shown, the graph 300 represents a comparison of a unipolar signal 302, an action potential signal 304, and a bipolar signal 306. As an example, a model (e.g., AI model) may be trained using one or more of the unipolar intracardiac signals 302, a bipolar intracardiac signals 306, or multipolar intracardiac signals and the action potential signal 304. The example action potential signal 304 illustrates a spike in potential followed by a decay in potential. A repolarization time and/or ARP may be determined from the action potential signal 304. The repolarization time and/or ARP may be compared to the one or more of the unipolar intracardiac signals 302, a bipolar intracardiac signals 306, or multipolar intracardiac signals over the same time frame to determine a correlation between the signals. As such, patterns in the one or more of unipolar intracardiac signals 302, bipolar intracardiac signals 306, or multipolar intracardiac signals may be correlated with the action potential signal 302, and particularly timing characteristics of the action potential such as the ARP or repolarization timing. A model, such as an AI model may be trained/tested using datasets including data indicative of the graph 300. By developing an AI model in this way, the model may then receive one or more of unipolar intracardiac signals, bipolar intracardiac signals, or multipolar intracardiac signals without the benefit of the action potential, and may still predict a reliable ARP or other characteristics such as the features or morphology of the AP waveform.

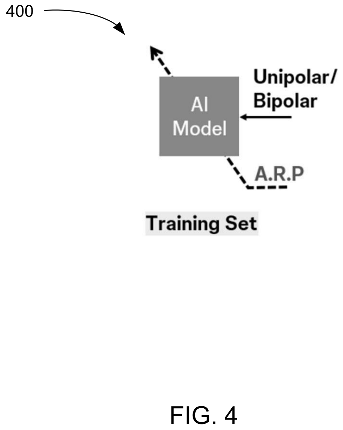

FIG. 4 shows an example method 400 for training an example artificial intelligence (AI) model disclosed herein. The example method 400 may comprise training the AI model on unipolar and/or bipolar intracardiac signals and known ARP. The known ARP may be determined by cell activity associated with the heart model and/or may include action potential data (e.g., simulated or measured). As an illustrative example, cells within a defined proximity of an electrode (e.g., 3mm radius from electrode center may be investigated and measured, or simulated. Data used to train the AI model may be augmented to simulate real conditions. Augmenting the data used to train the AI model may comprise adding real noise to the data and/or adding ventricle far field signals to the data. The AI model may be configured to determine repolarization timing (ARP) based on input of one or more unipolar and/or bipolar intracardiac signal(s).

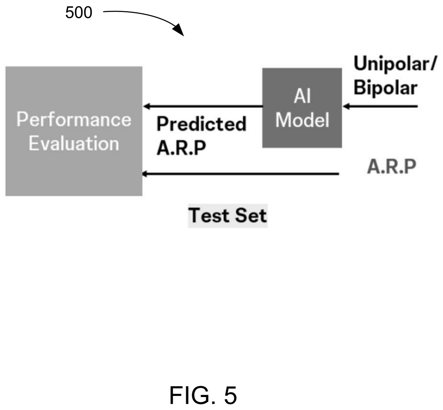

FIG. 5 shows an example method 500 for testing an example artificial intelligence (AI) model disclosed herein. The example method 500 may test the AI model trained by the example method 400 described in FIG. 4. The example method 500 may comprise inputting a dataset of unipolar and/or bipolar intracardiac signal(s) into the AI model and predicted ARP may be received as output from the AI model. The example method 500 may comprise comparing the predicted ARP with actual ARP to determine a performance evaluation associated with the AI model. Other outputs may be provided from the trained AI model. For example, the morphology of the AP waveform may be trained against other parameters such the unipolar, bipolar, multipolar signals such that predicted characteristics of an AP morphology may be outputted from the trained AI model.

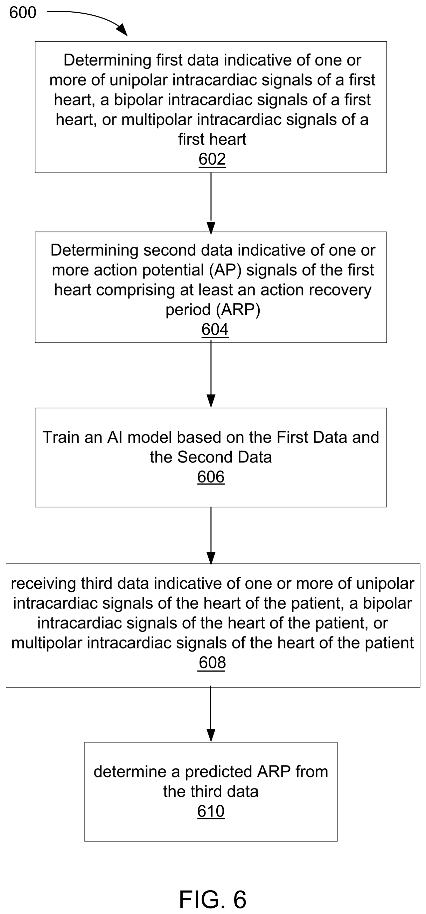

FIG. 6 illustrates a flowchart of a method 600 for using an artificial intelligence (AI) model to determine a repolarization wave in data from a model heart. One or more processors may perform the method 600.

First data may be determined at 602. The first data may be indicative of one or more of unipolar intracardiac signals of a heart, a bipolar intracardiac signals of a heart, or multipolar intracardiac signals of a heart. The first data may be simulated or may be measured. The first data may be simulated from a model of a heart, as described further herein.

Second data may be determined at 604. The second data may be indicative of one or more action potential signals of a heart comprising an action recovery period (ARP). The second data may comprise a repolarization wave. The second data may be simulated or may be measured. The second data may be simulated from a model of a heart, as described further herein.

An artificial intelligence (AI) model may be trained at 606. The AI model may be trained based on the first data and the second data. The AI model may be configured to determine a predicted ARP based on one or more of unipolar intracardiac signals of a heart, a bipolar intracardiac signals of a heart, or multipolar intracardiac signals of a heart. As described herein, the AI model may be trained/tested to determine a predicted ARP based only on unipolar intracardiac signals of a heart, a bipolar intracardiac signals of a heart, or multipolar intracardiac signals of a heart.

A third data may be received at 608. The third data may be indicative of one or more of unipolar intracardiac signals of a heart, a bipolar intracardiac signals of a heart, or multipolar intracardiac signals of a heart. The third data may be simulated or measured. As an example, third data may be measured from a physical heart of a patient.

Using the AI model, a predicted ARP may be determined from the third data at 610. By developing an AI model in this way, the model may then receive one or more of unipolar intracardiac signals, bipolar intracardiac signals, or multipolar intracardiac signals without the benefit of the action potential, and may still predict characteristics such as a reliable ARP, AP, or characteristic of the AP such as the morphology of the waveform. Other characteristics may be predicted using the AI model including, for example, various predicted, measured, or extracted attributes.

Although example blocks are shown, some implementations may include additional blocks, fewer blocks, different blocks, or differently arranged blocks than those depicted. Additionally, or alternatively, two or more of the blocks may be performed in parallel.

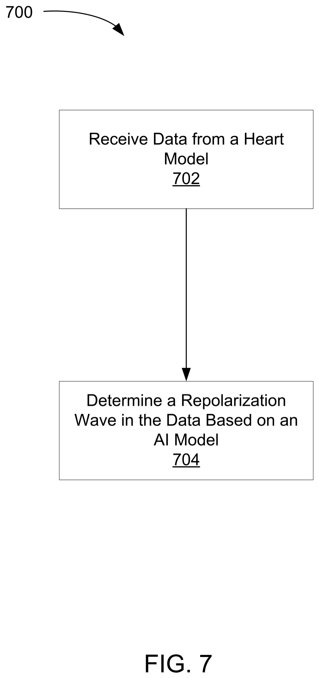

FIG. 7 illustrates a flowchart of a method 700 for using an artificial intelligence (AI) model to determine a repolarization wave in data from a model heart. One or more processors may perform the method 700. Data may be received from a heart model (block 702). The data may be simulated data. In one example, the heart model may comprise over ten million cells. However, any number of cells may be represented in the heart model. The heart model may comprise a combination of a physiological model, an anatomical model, and a physical model. The data may comprise one or more intracardiac signals. The data may comprise one or more unipolar signals. The data may comprise one or more bipolar signals. A repolarization wave may be determined in the data based on the AI model (block 704). The AI model may be evaluated (e.g., verified, checked, confirmed, etc.) by comparing the determined repolarization wave against an associated known repolarization wave. The AI model may have been trained (e.g., finetuned, etc.) on one or more datasets that associate repolarization waves with data from one or more heart models, such as the heart model.

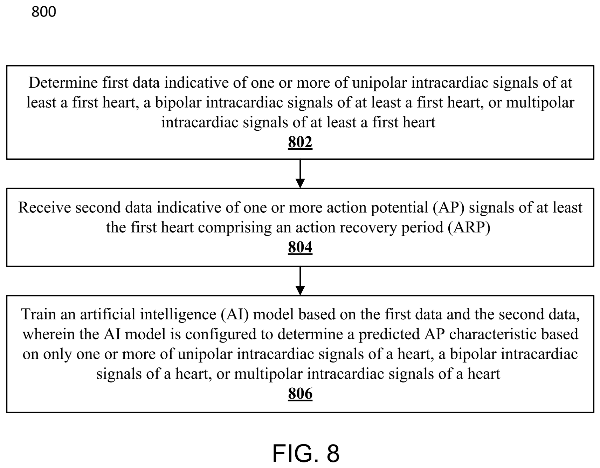

Fig. 8 is a flowchart of an example process 800. In some implementations, one or more process blocks of Fig. 8 may be performed by a device or devices.

As shown in Fig. 8, process 800 may include determining first data indicative of one or more of unipolar intracardiac signals of at least a first heart, a bipolar intracardiac signals of at least a first heart, or multipolar intracardiac signals of at least a first heart (block 802). For example, device may determine first data indicative of one or more of unipolar intracardiac signals of at least a first heart, a bipolar intracardiac signals of at least a first heart, or multipolar intracardiac signals of at least a first heart, as described above.

As also shown in Fig. 8, process 800 may include receiving second data indicative of one or more action potential (AP) signals of at least the first heart having an action recovery period (ARP) (block 804). For example, device may receive second data indicative of one or more action potential (AP) signals of at least the first heart having an action recovery period (ARP), as described above.

As further shown in Fig. 8, process 800 may include training an artificial intelligence (AI) model based on the first data and the second data, where the AI model is configured to determine a predicted AP characteristic based on only one or more of unipolar intracardiac signals of a heart, a bipolar intracardiac signals of a heart, or multipolar intracardiac signals of a heart (block 806). For example, device may train an artificial intelligence (ai) model based on the first data and the second data, where the ai model is configured to determine a predicted ap characteristic based on only one or more of unipolar intracardiac signals of a heart, a bipolar intracardiac signals of a heart, or multipolar intracardiac signals of a heart, as described above.

Although Fig. 8 shows example blocks of process 800, in some implementations, process 800 may include additional blocks, fewer blocks, different blocks, or differently arranged blocks than those depicted in Fig. 8. Additionally, or alternatively, two or more of the blocks of process 800 may be performed in parallel.

Although example blocks are shown, some implementations may include additional blocks, fewer blocks, different blocks, or differently arranged blocks than those depicted. Additionally, or alternatively, two or more of the blocks may be performed in parallel.

### EXAMPLES

The present disclosure comprises at least the following examples:
Example Clause A: A method for determining one or more predicted action potential characteristics of a heart of a patient, the method may include: determining first data indicative of one or more of unipolar intracardiac signals of a first heart, a bipolar intracardiac signals of a first heart, or multipolar intracardiac signals of a first heart; determining second data indicative of one or more action potential (AP) signals of the first heart may include at least an action recovery period (ARP); training an artificial intelligence (AI) model based on the first data and the second data, where the AI model is configured to determine a predicted ARP based on one or more of unipolar intracardiac signals of the first heart, a bipolar intracardiac signals of the first heart, or multipolar intracardiac signals of the first heart; receiving third data indicative of one or more of unipolar intracardiac signals of the heart of the patient, a bipolar intracardiac signals of the heart of the patient, or multipolar intracardiac signals of the heart of the patient; and determine, by the AI model and based on the third data, a predicted ARP of the heart of the patient.
Example Clause B: The method of Example Clause A, where the first data is simulated data.
Example Clause C: The method of Example Clause A or Example Clause B, where the first data may include simulated data.
Example Clause D: The method of any one of Example Clauses A-C, where the first data may include simulated data generated from at least a portion of a simulated heart model.
Example Clause E: The method of any one of Example Clauses A-D, where the heart model may include a combination of a physiological model, an anatomical model, and a physical model.
Example Clause F: The method of any one of Example Clauses A-E, where the second data is simulated data.
Example Clause G: The method of any one of Example Clauses A-F, where the second data may include simulated data.
Example Clause H: The method of any one of Example Clauses A-G, where the second data may include simulated data generated from at least a portion a simulated heart model.
Example Clause I: The method of any one of Example Clauses A-H, where the first data and the second data are represent disparate signals over the same period of time.
Example Clause J: The method of any one of Example Clauses A-I, where the third data is measured from the heart of the patient.
Example Clause K: A method for determining one or more predicted action potential characteristics of a heart of a patient, the method may include: determining first data indicative of one or more of unipolar intracardiac signals of at least a first heart, a bipolar intracardiac signals of at least a first heart, or multipolar intracardiac signals of at least a first heart; receiving second data indicative of one or more action potential (AP) signals of at least the first heart may include an action recovery period (ARP); training an artificial intelligence (AI) model based on the first data and the second data, where the AI model is configured to determine a predicted AP characteristic based on only one or more of unipolar intracardiac signals of a heart, a bipolar intracardiac signals of a heart, or multipolar intracardiac signals of a heart.
Example Clause L: The method of Example Clause K, where the first data is simulated data.
Example Clause M: The method of Example Clause K or Example Clause L, where the first data may include simulated data.
Example Clause N: The method of any one of Example Clauses K-M, where the first data may include simulated data generated from at least a portion of a simulated heart model.
Example Clause O: The method of any one of Example Clauses K-N, where the heart model may include a combination of a physiological model, an anatomical model, and a physical model.
Example Clause P: The method of any one of Example Clauses K-O, where the second data is simulated data.
Example Clause Q: The method of any one of Example Clauses K-P, where the second data may include simulated data.
Example Clause R: The method of any one of Example Clauses K-Q, where the second data may include simulated data generated from at least a portion of a simulated heart model.
Example Clause S: The method of any one of Example Clauses K-R, where the predicted AP characteristic may include one or more of a predicted ARP or a predicted morphology of an AP waveform.
Example Clause T: A method for determining one or more predicted action potential characteristics of a heart of a patient, the method may include: receiving simulated data from at least a portion of a heart model; and determine, using an artificial intelligence (AI) model and based on the simulated data, a repolarization wave indicative of a predicted action recovery period (ARP) of a heart of a patient.
Example Clause U: The method of Example Clause T, where the heart model may include a combination of a physiological model, an anatomical model, and a physical model.
Example Clause V: The method of Example Clause T or Example Clause U, where the simulated data may include one or more intracardiac signals.
Example Clause W: The method of any one of Example Clauses T-V, where the simulated data may include one or more unipolar signals.
Example Clause X: The method of any one of Example Clauses T-W, where the simulated data may include one or more bipolar signals.

The foregoing disclosure provides illustration and description but is not intended to be exhaustive or to limit the implementations to the precise form disclosed. Modifications may be made in light of the above disclosure or may be acquired from practice of the implementations. As used herein, the term "component" is intended to be broadly construed as hardware, firmware, or a combination of hardware and software. It will be apparent that systems and/or methods described herein may be implemented in different forms of hardware, firmware, and/or a combination of hardware and software. The actual specialized control hardware or software code used to implement these systems and/or methods is not limiting of the implementations. Thus, the operation and behavior of the systems and/or methods are described herein without reference to specific software code - it being understood that software and hardware can be used to implement the systems and/or methods based on the description herein. As used herein, satisfying a threshold may, depending on the context, refer to a value being greater than the threshold, greater than or equal to the threshold, less than the threshold, less than or equal to the threshold, equal to the threshold, and/or the like, depending on the context. Although particular combinations of features are recited in the claims and/or disclosed in the specification, these combinations are not intended to limit the disclosure of various implementations. In fact, many of these features may be combined in ways not specifically recited in the claims and/or disclosed in the specification

Although each dependent claim listed below may directly depend on only one claim, the disclosure of various implementations includes each dependent claim in combination with every other claim in the claim set. No element, act, or instruction used herein should be construed as critical or essential unless explicitly described as such. Also, as used herein, the articles "a" and "an" are intended to include one or more items and may be used interchangeably with "one or more." Further, as used herein, the article "the" is intended to include one or more items referenced in connection with the article "the" and may be used interchangeably with "the one or more." Furthermore, as used herein, the term "set" is intended to include one or more items (e.g., related items, unrelated items, a combination of related and unrelated items, and/or the like), and may be used interchangeably with "one or more." Where only one item is intended, the phrase "only one" or similar language is used. Also, as used herein, the terms "has," "have," "having," or the like are intended to be openended terms. Further, the phrase "based on" is intended to mean "based, at least in part, on" unless explicitly stated otherwise. Also, as used herein, the term "or" is intended to be inclusive when used in a series and may be used interchangeably with "and/or," unless explicitly stated otherwise (e.g., if used in combination with "either" or "only one of").

## Claims

1. A method for determining one or more predicted action potential characteristics of a heart of a patient, the method comprising:
determining first data indicative of one or more of unipolar intracardiac signals of a first heart, a bipolar intracardiac signals of a first heart, or multipolar intracardiac signals of a first heart;
determining second data indicative of one or more action potential (AP) signals of the first heart comprising at least an action recovery period (ARP);
training an artificial intelligence (AI) model based on the first data and the second data, wherein the AI model is configured to determine a predicted ARP based on one or more of unipolar intracardiac signals of the first heart, a bipolar intracardiac signals of the first heart, or multipolar intracardiac signals of the first heart;
receiving third data indicative of one or more of unipolar intracardiac signals of the heart of the patient, a bipolar intracardiac signals of the heart of the patient, or multipolar intracardiac signals of the heart of the patient; and
determine, by the AI model and based on the third data, a predicted ARP of the heart of the patient.

2. The method of claim 1, wherein the first data is simulated data.

3. The method of claim 1 or claim 2, wherein the first data comprises simulated data.

4. The method of any one of claims 1 to 3, wherein the first data comprises simulated data generated from at least a portion of a simulated heart model.

5. The method of any one of claims 1 to 4, wherein the heart model comprises a combination of a physiological model, an anatomical model, and a physical model.

6. The method of any one of claims 1 to 5, wherein the second data is simulated data.

7. The method of any one of claims 1 to 6, wherein the second data comprises simulated data.

8. The method of any one of claims 1 to 7, wherein the second data comprises simulated data generated from at least a portion a simulated heart model.

9. The method of any one of claims 1 to 8, wherein the first data and the second data are represent disparate signals over the same period of time.

10. The method of any one of claims 1 to 9, wherein the third data is measured from the heart of the patient.

11. A method for determining one or more predicted action potential characteristics of a heart of a patient, the method comprising:
determining first data indicative of one or more of unipolar intracardiac signals of at least a first heart, a bipolar intracardiac signals of at least a first heart, or multipolar intracardiac signals of at least a first heart;
receiving second data indicative of one or more action potential (AP) signals of at least the first heart comprising an action recovery period (ARP);
training an artificial intelligence (AI) model based on the first data and the second data, wherein the AI model is configured to determine a predicted AP characteristic based on only one or more of unipolar intracardiac signals of a heart, a bipolar intracardiac signals of a heart, or multipolar intracardiac signals of a heart.

12. The method of claim 11, wherein the first data comprises simulated data generated from at least a portion of a simulated heart model.

13. The method of claim 11 or claim 12, wherein the heart model comprises a combination of a physiological model, an anatomical model, and a physical model.

14. The method of any one of claims 11 to 13, wherein the second data comprises simulated data generated from at least a portion of a simulated heart model.

15. The method of any one of claims 11 to 14, wherein the predicted AP characteristic comprises one or more of a predicted ARP or a predicted morphology of an AP waveform.
